# EUROPEAN PATENT APPLICATION

(11) **EP 4 485 475 A1**
(43) Date of publication of application: **01.01.2025**
(21) Application number: 22928889.9
(22) Date of filing: 17.11.2022
(51) Int. Cl.: G16H 40/20

(54) **EMERGENCY SICK-AND-WOUNDED CARE SYSTEM**

(30) Priority: 22.02.2022 JP 2022025389
(71) Applicant: Nippon BXI Incorporated, Tokyo 160-0003 (JP)
(72) Inventor: TANI Kazuo, Tokyo 160-0003 (JP); TSUKAHARA Yukitoshi, Nagoya-shi, Aichi 460-0026 (JP)
(74) Representative: McWilliams, David John
(86) International application number: PCT/JP2022/042678
(87) International publication number: WO 2023/162372

(57) **Abstract**

A system comprises a device for reading private information configured to read private information of an injured or sick person, a device for reporting injured and sick person configured to report condition of the person to a family doctor based on family doctor information included in the private information, a device for giving treatment direction configured to give treatment direction to an ambulance whether the family doctor will treat the person by him or herself or request treatment to a local doctor, a device for requesting treatment configured to request treatment to the local doctor when treatment is requested to the local doctor, a clinical history database storing clinical history for each individual, and a device for extracting and transmitting clinical history data configured to extract clinical history data based on the private information and send the data to the local doctor when treatment is requested to the local doctor.

## Description

### [TECHNICAL FIELD]

The present disclosure relates to an emergency treatment system for injured or sick persons.

### [BACKGROUND]

According to Japanese Unexamined Patent Application Publication No. 2002-269662, a system is disclosed in which the system is configured to warn a person when the system detects an abnormality in biological information of the person, and then the system provides a center with locational information and/or user ID of the person.

When the person loses consciousness, information such as an abnormality and the location of the person is notified to a center. However, in that case, it takes time to start appropriate treatment for the person after the center receives a notification.

Accordingly, an improved emergency system is required.

### [SUMMARY]

In a first aspect of the present disclosure, an emergency treatment system for an injured and/or sick person comprises a database about a family doctor or hospital, a means for reading private information, a means for extracting family doctor, a means for reporting injured and sick person, a means for giving treatment direction, a means for requesting treatment, a clinical history database, and a means for extracting and transmitting clinical history data. The database stores family doctors previously determined for each individual. The means for reading private information is configured to read private information of an injured or sick person by an operation of a paramedic. The means for extracting family doctor is configured to extract a family doctor from the database based on the private information. The means for reporting injured and sick person is configured to report the condition of the person to the family doctor. The means for giving treatment direction is configured to give treatment direction to the ambulance based on the report from the means for reporting injured and sick person whether the family doctor will treat the person by him or herself or request treatment to a local doctor who is in an area where the person is. The means for requesting treatment is configured to request treatment to the local doctor by an operation of the paramedic when the treatment direction given by the means for giving treatment direction is that treatment of the person is requested to the local doctor. The clinical history database stores clinical history previously registered in advance for each individual. The means for extracting and transmitting clinical history data is configured to extract clinical history data of the person from the clinical history database and send the clinical history data to the local doctor as reference data for treatment based on the private information when treatment of the person is requested to the local doctor via the means for requesting treatment.

According to the first aspect, if a person gets sick and/or is injured while traveling or going out, a family doctor of the person can be specified in an ambulance based on the private information of the person. The family doctor can determine an appropriate treatment direction depending on the situation after receiving a report from the ambulance. Then, the ambulance can transport the person to the family doctor or a local doctor based on the treatment direction. When the local doctor treats the person, clinical history data about the person will be sent to the local doctor. In this way, the person can receive appropriate treatment based on the determination of the family doctor without wasting time, even when the person is traveling or going out. That is, the person who was taken to the hospital by ambulance can receive appropriate and quick treatment, depending on the clinical history of the person.

In a second aspect of the present disclosure, the private information, which is read by the means for reading private information, includes biological information, such as an electrocardiogram, heart rate, and body temperature at that time. The report from the means for reporting injured and sick person and the request from the means for requesting treatment may further include biological information.

According to the second aspect, the report from the means for reporting injured and sick person includes biological information about the person at that time. The request from the means for requesting treatment also includes the biological information of the person. Thus, the biological information can be utilized to determine the treatment direction of the family doctor and to treat the person by the local doctor.

In a third aspect of the present disclosure, the system includes a family database, a means for extracting a family, a means for reporting to a family, and a means for transmitting family's intention. The family database stores family data which is previously registered as a family who receive the report. The means for extracting a family is configured to extract from the family database a family who receives the report based on the private information read by the means for reading private information. The means for reporting to a family is configured to report the condition and location of the person to the family by an operation of the crew of ambulance. The means for transmitting family's intention is configured to transmit family's intention to the family doctor that the treatment of the person is requested to the family doctor or the local doctor after receiving the report from the means for reporting to a family.

According to the third aspect, the family's intention whether the treatment of the person is requested to the family doctor or the local doctor is transmitted to the family doctor. Thus, the family doctor can determine the treatment direction based on the family's intention.

In a fourth aspect of the present disclosure, the system includes a means for inputting location and injured and sick condition, a local doctor database, and a means for extracting local doctor. The means for inputting location and injured and sick condition is configured to input the location and condition of the person by an operation of the crew of the ambulance. The system includes a local doctor database storing the names of medical specialists by region and injured or sick conditions. The system includes a means for extracting local doctor configured to extract local doctors available at the location from the local doctor database based on the location and condition of the person input by the means for inputting location and injured and sick condition, and then provide the family doctor who determines the treatment direction with information.

According to the fourth aspect, the family doctor can refer to local doctors information when the family doctor determines the treatment direction. Thus, the family doctor can appropriately determine the treatment direction.

In a fifth aspect of the present disclosure, the system includes a means for notifying transportation configured to notify the family doctor by an operation of the crew of an ambulance that the person will be transported to the family doctor when the treatment direction is that the family doctor treats the person by him or herself.

According to the fifth aspect, when the treatment direction is that the family doctor treats the person by him or herself, the means for notifying transportation notifies that the person will be transported. Thus, the family doctor can appropriately prepare for reception of the person.

### [BRIEF DESCRIPTION OF THE DRAWINGS]

[FIG. 1] FIG. 1 is a system configuration figure according to an embodiment of the present disclosure.
[FIG. 2] FIG. 2 is a functional block diagram according to the embodiment.
[FIG. 3] FIG. 3 is a flow chart showing the program content of a computer mounted on an ambulance according to the embodiment.
[FIG. 4] FIG. 4 is a flow chart showing the program content of a computer owned by a family according to the embodiment.
[FIG. 5] FIG. 5 is a flow chart showing the program content of a computer owned by a family doctor according to the embodiment.
[FIG. 6] FIG. 6 is a flow chart showing the program content of a computer owned by a local doctor who is in an actual place according to the embodiment.

### [MODES FOR CARRYING OUT THE INVENTION]

### < A system configuration according to the embodiment>

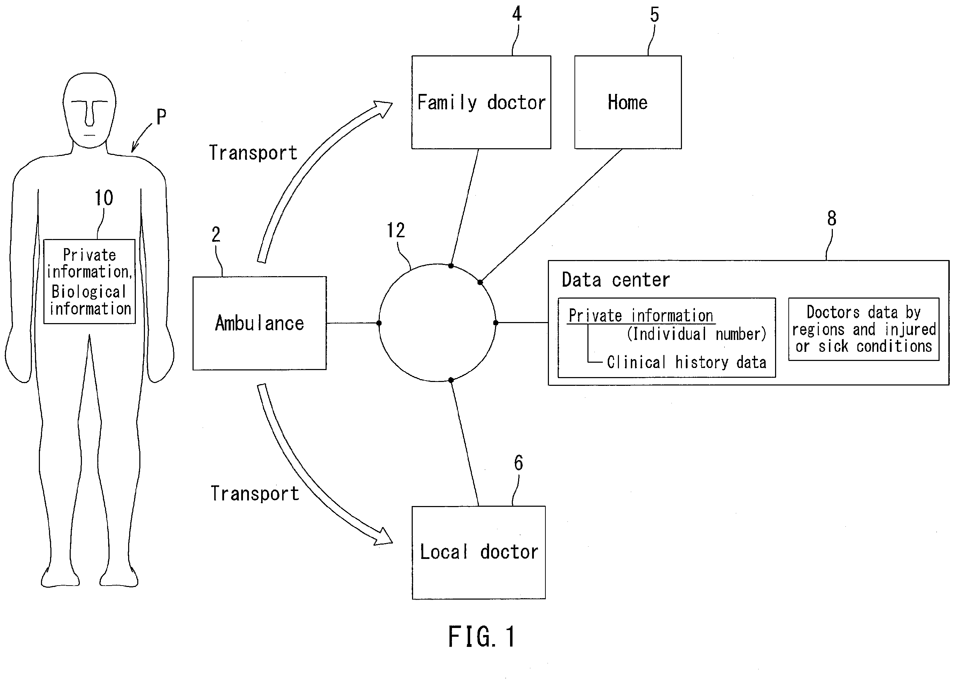

Figure 1 shows a system configuration of an emergency treatment system for injured or sick persons according to an embodiment. A person who uses this system has an implantable microchip 10 (hereinafter "chip") in the body. The chip 10 may be a portable storage device, a smartwatch, or a sensor attachable to a body surface, instead of the implantable one. For example, the sensor may be adhered to a body surface with tape or an adhesive agent, or the sensor may be fixed to a body surface by a belt. The chip 10 stores private information and biological information at present time and in the former period of that time. For example, the former period may be about 1 hour. The private information can be updated by an information rewriter (not shown) operable outside of the body as required. Also, the biological information can be updated by responding to detection signals from an implantable vital sensor or a vital sensor attachable to a body surface (not shown). Examples of private information and biological information are as follows.

### <Examples of private information >

Health information: Identity number, a record of medical examinations (a clinical record), health insurance card information, driving license information, information about a family doctor, information of family, clinical history (including electronic clinical record), medication record, etc.

The other information: passport information, visa information, military service record, seminar participation record, criminal record, kidnapper record, owing weapon record, dementia record, drone operator's license information, nationality (colors of skin, hair, eyes), location information (from GPS). etc.

For example, biological information may include images and examination records recorded in examination machines, such as an implantable defibrillator, a pacemaker, an X-ray examination machine, and MRI, in addition to an electrocardiogram, heart rate, body temperature, blood pressure, and SpO₂ (oxygen saturation).

When a person has a chip 10, even if the person P lost consciousness due to disease and/or an injury outside or at a place where the person stays, the person can be easily identified by an aiding person having an information reader. An ambulance 2, which transports the person P to a hospital, may have a reader (not shown) to read information stored in the chip 10. When the chip 10 stores location information and has an emergent communication function, it is possible to identify a place where the injured and/or sick person P met with a disaster. Even if the person P stays on a remote island or in a remote place, identification of the place is also possible. Further, if there is a web environment in that place, the person P can take medical advice on the web. An ambulance 2 can be connected to a computer network 12 via mobile communication and/or a wireless LAN (local area network). Computers of doctors 4 and 6, and a computer located in home 5 (not shown) are connected to the computer network 12 via a LAN connection. The doctors 4 and 6, and home 5 are registered in advance as users of an emergency treatment system for injured or sick persons. A data center 8 is connected to the computer network 12 via a LAN connection. A storage device in the data center 8 stores examination results and information to identify a clinical history (including medication records) by responding to private information (especially an identity number). Thus, for example, personal clinical history can be read out from private information. The examination result may include results of physiological examination (e.g., electrocardiogram, blood pressure, heart rate, SpO₂, and body temperature), blood examination (e.g., hematological examination, biochemical examination), image examination (e.g., X-ray examination, MRI examination, an ultrasonic diagnosis result, and a proton beam therapy result). A clinical history includes a result of Al analysis. The storage device in the data center 8 stores information of medical specialists by injuries and diseases and by region. Thus, it is possible to select a medical specialist at the location of an injured and/or sick person P based on the location and condition of the person P. The region is not limited to domestic areas and includes those abroad. An ambulance 2 may be an emergency hospital.

### <Functions according to an embodiment>

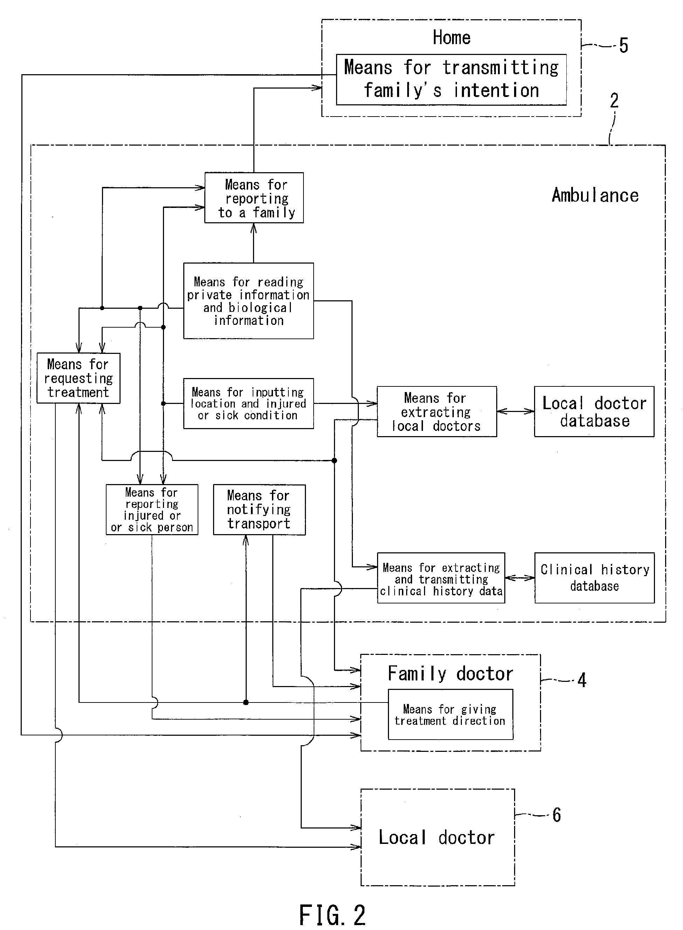

Figure 2 shows the functions of the emergency treatment system for injured or sick persons as function blocks. As shown in Figure 2, in a means (device) for reading private information and biological information provided in an ambulance 2, a crew of the ambulance 2 can operate a reader to read private information and biological information stored in a chip 10. The reader may be, for example, a microchip reader, a smartphone, a smartwatch, a cell phone, or a (QR) code reader. Based on family information, which is included in private information read by the means for reading private information and biological information, a family of an injured and/or sick person P is extracted. The family is registered in advance as a family that receives a report when the person P gets injured and/or sick. Further, based on family doctor information, which is included in the private information, a family doctor of the person P is extracted. The family doctor is registered in advance as a family doctor that receives a report when the person P gets injured and/or sick. Alternatively, the family doctor may be a hospital with which the person P contracts in advance. Further, based on the private information read by the means for reading private information and biological information, in a means (device) for extracting and transmitting clinical history data, clinical history data of the person P is extracted from clinical history database stored in the storage device of the data center 8, together with examination results. The device for extracting and transmitting clinical history data may be, for example, a PC, a smartphone, a tablet terminal, a smartwatch, a cell phone, and a (QR) code reader. The clinical history data has the same content as data of clinical records made by doctors who diagnosed and treated the person P previously. The clinical history data is transmitted to a local doctor, as described later.

In a means (device) for inputting location and injured and sick condition, a crew of the ambulance 2 inputs location and injured and sick condition of the person P. Based on the information on the location and the injured and sick condition of the person P, in a means (device) for extracting local doctor, a medical specialist is extracted who can diagnose and treat the person P at the location where the person P stays from a local doctor database stored in the storage device of the data center 8. The device for inputting location and injured and sick condition and the device for extracting local doctor may be, for example, a PC, a smartphone, a tablet terminal, a smartwatch, a cell phone, and a (QR) code reader. A plurality of doctors may be extracted in order to increase possibility of finally requesting.

The personal information read by the means for reading private information and biological information and the location and condition of the person P input via the means for inputting location and injured and sick condition are reported to the family read via a means (device) for reporting to a family. The family who received the report may communicate with the family doctor about whether the family wishes to request treatment of the person P to the family doctor or a local doctor who is in the area where the person P stays via a means (device) for transmitting family's intention. On the other hand, the location and the condition of the person P are reported to the family doctor via a means (device) for reporting injured and sick person. In addition, biological information read by the means for reading private information and biological information is also reported to the family doctor via the means for reporting injured and sick person. After receiving the report, the family doctor gives direction to the crew of the ambulance 2 on whether the family doctor treats the person P by him or herself or requests treatment to the local doctor, via a means (device) for giving treatment direction. In order to decide the treatment direction, the family doctor may refer to information provided by the means for reporting injured and sick person, information of local doctors extracted by the means for extracting local doctor, and the family's intention with respect to the treatment direction transmitted by the means for transmitting family's intention. The device for reporting to family, the device for transmitting family's intention, the device for reporting injured and sick person, and the device for giving treatment direction may be, for example, a PC, a smartphone, a tablet terminal, a smartwatch, a cell phone, and a (QR) code reader.

The ambulance 2 informs the family doctor 4 via a means (device) for notifying transportation that the person P will be transported to the family doctor when the treatment direction, which is given via the means for giving treatment direction, is that the family doctor treats the person P by him or herself. On the other hand, when the treatment direction is that the treatment of the person P is requested to a local doctor, the ambulance 2 requests the local doctor 6 to treat the person P via a means (device) for requesting treatment. When treatment is requested to the local doctor 6, biological information read by the means for reading private information and biological information and the information on the condition of the person P read by the means for inputting location and injured and sick condition are also sent. A clinical history including past examination results and medication records of the person P who is requested to be treated via the means for extracting and transmitting clinical history data as described above is provided to the local doctor 6 as reference data for treatment. Thus, the local doctor 6 can properly treat the person P at a location near the person P without wasting time. Optionally, the local doctor 6 may request a second opinion of the family doctor 4 about the treatment of the person P. The device for notifying transportation and the device for requesting treatment may be, for example, a PC, a smartphone, a tablet terminal, a smartwatch, a cell phone, or a (QR) code reader.

### <Processing content of a computer in an ambulance>

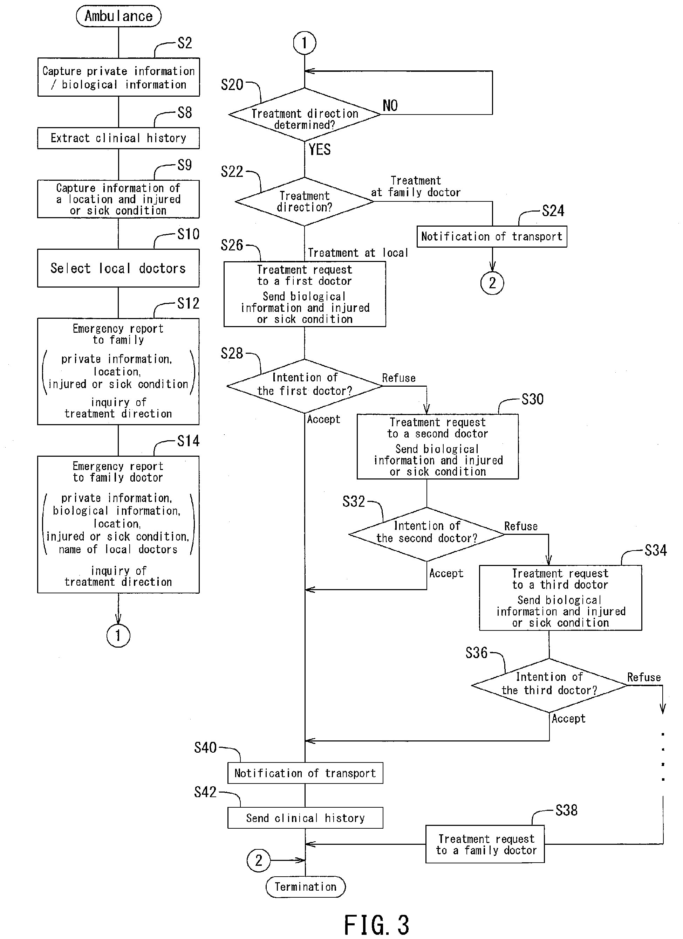

Figure 3 shows the contents of a processing program carried out by a computer mounted on the ambulance 2 to achieve the functions described in Figure 2. At the step S2, private information and biological information read from the chip 10 by the crew of the ambulance 2 with a reader are captured. The private information captured at the step S2 may include family information and family doctor information. At the step S8, clinical history is extracted from a clinical history database based on the private information captured at the step S2.

At the step S9, information on the location and injured and sick condition of the person P input by the crew of the ambulance 2 are captured. At the next step S 10, a plurality of medical specialists who can diagnose and treat the person P at a place near the person P is selected from the local doctor database based on the information on the location and injured and sick condition of the person P captured at the step S9.

At the step S12, information of the person P is reported to the family extracted at the step S4. The report includes the private information captured at the step S2, the location and the injured and sick condition of the person P captured at the step S9. At the step S12, a request of the family about a way of treatment is inquired.

At the next step S14, the information of the person P is reported to the family doctor who is captured at the step S2. The report includes the private information and the biological information captured at the step S2 and the location and injured and sick condition of the person P captured at the step S9. Further, the names of local doctors selected at the step S10 may be included in the report. At the step S14, treatment direction is also inquired.

At the step S20, it is determined whether the family doctor has notified treatment direction to the inquiry of treatment direction at the step S14. When the step S20 is affirmatively determined, the content of the treatment direction is determined at the step S22. When the treatment direction is that the family doctor treats, it is notified to the family doctor at the step S24 that the person P will be carried. When the treatment direction is that the local doctor is requested to treat, the treatment request is sent to a first doctor at the step S26 who is one of the local doctors. The local doctor is selected from the doctors who were selected at the step S10. The request to the first doctor includes the biological information captured at the step S2 and the injured and sick condition captured at the step S9 as reference information.

When intention of the first doctor is expressed against the request at the step S26, content of the intention is determined at the step S28. When the first doctor intends that the first doctor will accept the request, the first doctor is notified at the step S40 that the person P will be transported to the first doctor. On the other hand, when the result of the determination at the step S28 is that the first doctor did not accept and refused the request, a treatment request is sent to a second doctor at the step S30 who is one of the local doctors selected at the step S10 and is different from the first doctor. The treatment request to the second doctor includes the biological information and the injured and sick condition of the person P as well as the request to the first doctor.

When intention of the second doctor is expressed against the request at the step S30, the content of the intention is determined at the step S32. When the second doctor intends that the second doctor will accept the request, the second doctor is notified at the step S40 that the person P will be transported to the second doctor. On the other hand, when the result of the determination at the step S32 is that the second doctor did not accept and refused the request, a treatment request is sent to a third doctor at the step S34. The third doctor is one of the local doctors selected at the step S10 and is different from the first and the second doctors. The treatment request to the third doctor includes the biological information and the injured and sick condition of the person P as well as the request to the first and the second doctors.

When intention of the third doctor is expressed against the request at the step S34, the content of the intention is determined at the step S36. When the third doctor intends that the third doctor will accept the request, the third doctor is notified at the step S40 that the person P will be transported to the third doctor. On the other hand, when the result of the determination at the step S36 is that the third doctor did not accept and refused the request, a treatment request is sent to a fourth doctor and a fifth doctor who are the local doctors selected at the step S10. In this way, the same process may be repeated in a sequential order. When all treatment requests to the local doctors are unacceptable, a treatment request is sent to the family doctor.

When the treatment request to the local doctor is accepted and the notification of transport at the step S40 is done, data of clinical history of the person P, which was extracted at the step S8, is sent to the local doctor at the step S42 who accepted the treatment request.

### < Processing content by a computer of a family >

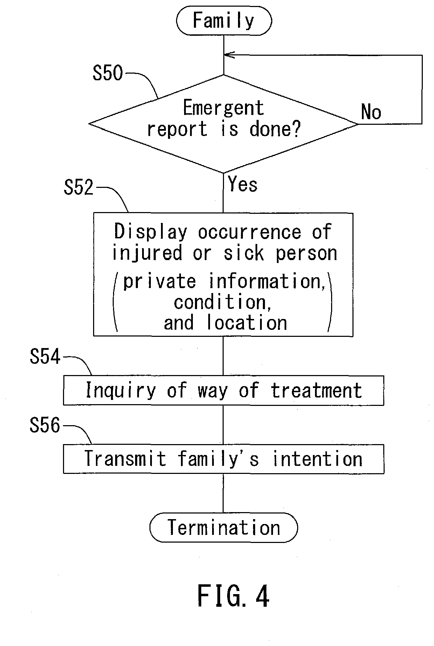

Figure 4 shows contents of processing program carried out by a computer of the home 5 to achieve functions described in Figure 2. At the step S50, it is confirmed whether an emergency report is done at the step S12 of Figure 3. When it is confirmed at the step S50 that the emergency report is done, occurrence of the injuries and sickness of the person P is displayed at the step S52. At this time, the private information, the location, and the condition of the injuries and sickness of the person P, which were sent at the step S12, are displayed. At the step S54, the inquiry about a way of treatment is received and displayed which has been sent at the step S12. At the step S56, the family's intention about the way of treatment is sent to respond to the inquiry at the step S54.

### <Processing content of a computer of a family doctor >

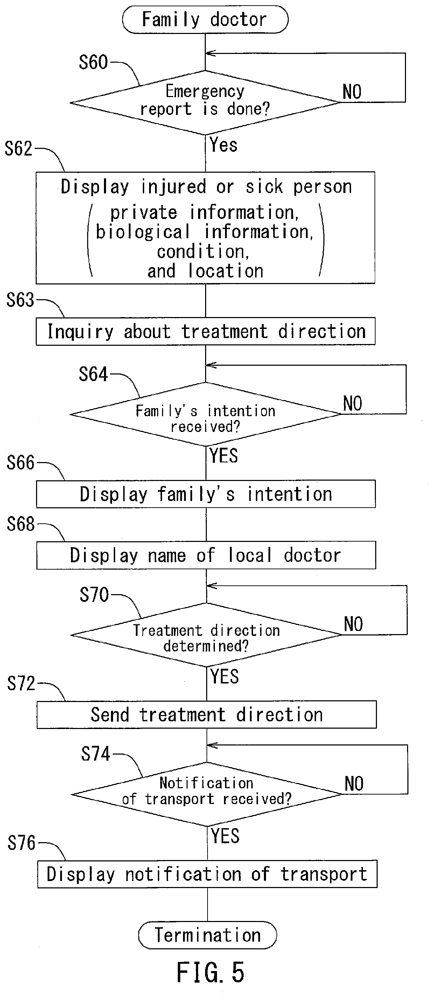

Figure 5 shows content of processing program which is carried out by a computer of a family doctor 4 to achieve the functions described in Figure 2. At the step S60, it is confirmed whether an emergency report is done at the step S14 of Figure 3. When it is confirmed at the step S60 that an emergency report is done, the occurrence of the injuries and sickness of the person P is displayed at the step S62. At this time, the private information, the biological information, the location, and the condition of the injuries and sickness of the person P, which were sent at the step S14, are displayed. At the step S63, the inquiry about treatment direction which has been sent at the step S14 is received and displayed.

At the step S64, it is determined whether the family's intention about the way of treatment is received. When the family's intention at the step S56 in Figure 4 is received, the step S64 is affirmatively determined and then, the family's intention is displayed at the step S66. Further, at the step S68, the names of the doctors selected at the step S10 are displayed. At the step S70, it is determined whether the family doctor determined treatment direction. When treatment direction is determined and the step S70 is affirmatively determined, the treatment direction is sent to the ambulance 2 at the step S72. At the step S74, it is determined whether the notification of transport at the step S24 is received. When the notification of transport is received and the step S 74 is affirmatively determined, the notification of transport is displayed at the step S76. With this display, the family doctor can prepare for receiving the person P who will be transported to the family doctor.

### <Processing content of a computer of a local doctor >

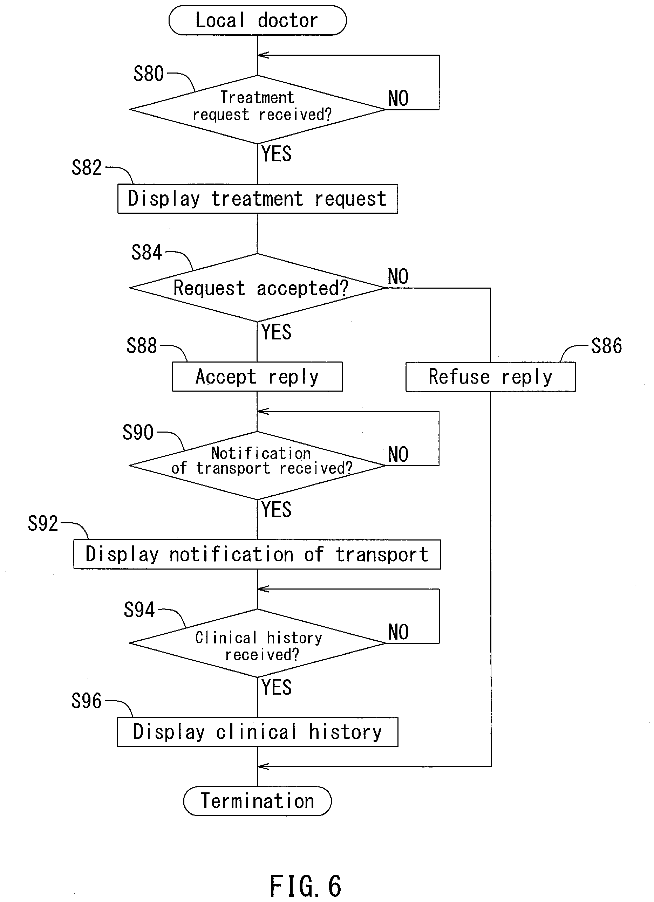

Figure 6 shows content of processing program which is carried out by a computer of a local doctor 6 to provide the functions described in Figure 2. At the step 80, it is determined whether the treatment request, which was sent at the steps S26, S30, and S34, has been received. When the treatment request is received, the step S80 is affirmatively determined. Then, the treatment request is displayed in the step S82. At the step S84, it is determined whether the local doctor will accept the treatment request. When the local doctor does not accept the treatment request, negative determination is made at the step S84 and then, a response that the treatment request is refused is sent to the ambulance 2 at the step S86. When the treatment request is accepted, the step S84 is affirmatively determined. Then, a response that the treatment request is accepted is sent to the ambulance 2 at the step S88.

After the response that the treatment request is accepted at the step S88, it is determined at the step S90 whether the notification of transport at the step S40 is received. When the notification of transport is received, the step S 90 is affirmatively determined and then, the notification of transport is displayed at the step S92. Further, it is determined at the step S94 whether the data of clinical history, which was sent at the step S42 in Figure 3, has been received. When the data on clinical history is received, the step S94 is affirmatively determined and then, the data on clinical history is displayed in the step S96.

There are following correspondence relation in the processes shown in Figures 3-6. The process of step S2 corresponds to the means for reading private information of the present disclosure. The process of step S14 corresponds to the means for reporting injured and sick person of the present disclosure. The process of step S72 corresponds to the means for giving treatment direction of the present disclosure.
The processes of steps S26, S30, and S34 correspond to the means for requesting treatment of the present disclosure. The processes of steps S8 and S42 correspond to the means for extracting and transmitting clinical history data of the present disclosure. The process of step S12 corresponds to the means for reporting to a family of the present disclosure.
The process of step S56 corresponds to the means for transmitting family's intention of the present disclosure. The process of step S9 corresponds to the means for inputting location and injured and sick condition of the present disclosure.
The process of step S10 corresponds to the means for extracting local doctors of the present disclosure. The process of step S24 corresponds to the means for notifying transportation of the present disclosure.

According to the above-mentioned embodiments of the emergency treatment system for injured or sick persons, if a person P gets sick and/or is injured while traveling or going out, a family doctor 4 of the person P can be specified in an ambulance 2 based on the private information of the person P. The family doctor 4 can determine an appropriate treatment direction depending on the situation after receiving a report from the ambulance 2. That is, the family doctor 4 can comprehensively determine the treatment direction whether the family doctor will treat the person P by him or herself or request treatment to a local doctor 6 based on the location and condition of the person P and the presence of the local doctor 6. The ambulance 2 can transport the person P to the family doctor 4 or the local doctor 6 based on the treatment direction. When the local doctor 6 treats the person P, clinical history data of the person P will be sent to the local doctor 6. In this way, the person P can receive appropriate treatment based on the determination of the family doctor 4 without wasting time even when the person P is traveling or going out.

### <The other embodiments>

Although specific embodiments are described above, the present disclosure is not limited to their appearance and/or structure. Rather, various modifications, additions, and deletions are available for the present disclosure. For example, in the above-mentioned embodiments, both the private information and the biological information of the person P are read out and then, the biological information is provided to the family doctor and the local doctor, however, the biological information is not necessarily needed. In the above-mentioned embodiments, the family doctor considers the family's intention when the family doctor determines treatment direction, however, the family's intention is not necessarily needed. In the above-mentioned embodiments, a list of local doctors is extracted from the database, however, an ambulance crew may manually select and determine a local doctor. In the above-mentioned embodiments, many local doctors are listed, however, only one local doctor may be selected. In the above-mentioned embodiments, a notification can be sent to the family doctor and the local doctor, however, such notification is not necessarily needed.

In the present disclosure, when an appropriate doctor cannot be found, hospitals located in different areas will be needed to cooperate. Further, it is noted that cooperation and/or assistance from medical associations may be needed. In some cases, paramedics may temporarily carry on the present disclosure.

In recent days, every person has a smartphone. Thus, smartphones may be dedicated devices to manage diseases of each person. Smartphones may be used to transmit and receive images such as an image of electrocardiograms, X-rays, and MRIs since smartphones have a high number of pixels. Teletherapy is available through the emergency system of the present disclosure by using the internet. In this way, emergency patients can utilize a global network. Although it is possible to install a dedicated application for the emergency system of the present disclosure on current smartphones, smartphones may be used as dedicated devices for the emergency system. Further, the dedicated devices may be made smaller so as to be implanted into bodies. This allows the health management of people via wireless communication. Thus, the emergency system of the present disclosure can be good news for patients, for example, who are suffering from lifestyle diseases. Furthermore, the emergency system of the present disclosure can be applied to preventive medicine.

Various embodiments described above by referring to the attached drawings are representative examples of the present disclosure and are not intended to limit the scope of the present disclosure. The detailed description teaches so that a person of ordinary skill in the art can make, use, and/or carry on various aspects of the present teachings. The detailed description is not intended to limit the scope of the present disclosure. Further, each of the additional characteristics and teachings described above may be used and/or applied separately or together with the other characteristics and teachings to provide an improved system.

## Claims

1. An emergency treatment system for an injured or sick person, the system comprising:
a device for reading private information being configured for an ambulance crew to read the private information of the injured or sick person;
a device for reporting injured and sick person being configured to report a condition of the person to a family doctor based on family doctor information included in the private information read by the device for reading private information;
a device for giving a treatment direction being configured to give the treatment direction to the ambulance based on a report from the device for reporting injured and sick person whether the family doctor will treat the person or request the treatment to a local doctor who is in an area where the person is;
a device for requesting the treatment being configured to request the treatment to the local doctor by an operation of the crew of the ambulance when the treatment direction given by the device for giving treatment direction is that treatment of the person is requested to the local doctor;
a clinical history database for storing clinical history data being registered in advance for each individual; and
a device for extracting and transmitting the clinical history data being configured to extract the clinical history data of the person from the clinical history database based on the private information read by the device for reading private information and send the clinical history data to the local doctor as reference data for treatment when treatment of the person is requested to the local doctor via the device for requesting treatment.

2. The emergency treatment system for an injured or sick person according to claim 1,
wherein the private information read by the device for reading private information includes biological information such as electrocardiogram, heart rate, and body temperature of the person, and
wherein the report from the device for reporting injured and sick person and request from the device for requesting treatment include the biological information as additional information.

3. The emergency treatment system for an injured or sick person according to claim 1 or 2, the system further comprising:
a device for reporting to family being configured to report the condition and location of the person to a family of the person by an operation of the crew of the ambulance; and
a device for transmitting family's intention being configured to transmit family's intention to the family doctor that the treatment of the person is requested to the family doctor or the local doctor after receiving the report from the device for reporting to family;
wherein the family is extracted from family information included in the private information, and the family is determined in advance to receive information on the person.

4. The emergency treatment system for the injured or sick person according to any one of claims 1 to 3 further comprising:
a device for inputting location and injured and sick condition being configured to input the location and condition of the person by the crew of the ambulance;
a local doctor database for storing names of medical specialists by region and injured or sick conditions,
a device for extracting local doctor being configured to extract local doctors available at the location from the local doctor database based on the location and condition of the person which is input by the device for inputting location and injured and sick condition, and provide the family doctor who determines the treatment direction with information of the local doctors.

5. The emergency treatment system for the injured or sick person according to any one of claims 1 to 4, further comprising a device for notifying transportation being configured to notify the family doctor by the ambulance crew that the person will be transported to the family doctor when the treatment direction is that the family doctor treats the person.
